# EUROPÄISCHE PATENTANMELDUNG

(11) **EP 1 094 062 A1**
(43) Veröffentlichungstag der Anmeldung: **25.04.2001**
(21) Anmeldenummer: 00116383.1
(22) Anmeldetag: 28.07.2000
(51) Int. Cl.: C07D 311/72, C07C 67/52

(54) **Verfahren zur Herstellung von veresterten Chromanverbindungen**

(30) Priorität: 22.10.1999 DE 19951006
(71) Anmelder: Degussa-Hüls Aktiengesellschaft, 60287 Frankfurt am Main (DE)
(72) Erfinder: Weigel, Horst, 63517 Rodenbach (DE); Krill, Steffen, Dr., 67346 Speyer (DE); Hasselbach, Hans Joachim, Dr., 63571 Gelnhausen (DE); Huthmacher, Klaus, Dr., 63584 Gelnhausen (DE)

(57) **Zusammenfassung**

Die Erfindung betrifft ein Verfahren zur Herstellung von veresterten Chromanverbindungen aus 2,6,6-Trimethylcylohex-2-en1,4-dion, wobei man die veresterten Zwischenprodukte ohne zusätzlichen Reinigungsschritt direkt zu den gewünschten Chromanderivaten umsetzt.

## Beschreibung

Die Erfindung betrifft ein Verfahren zur Herstellung von veresterten Chromanverbindungen aus in technischer Reinheit vorliegendem 2,6,6-Trimethylcyclohex-2-en1,4-dion (4-oxo-Isophoron, KIP), wobei man die veresterten Zwischenprodukte ohne zusätzlichen Reinigungsschritt direkt zu den gewünschten Chromanverbindungen umsetzt.

Die wichtigste Verbindung dieser Stoffgruppe ist das Vitamin E, das überwiegend als Ester im Handel ist. Chromanverbindungen sind im übrigen als Pharmazeutika, Futtermitteladditive und als Antioxidantien von Bedeutung.

Die Reaktion läuft für Vitamin E-Acetat nach folgendem Schema ab:

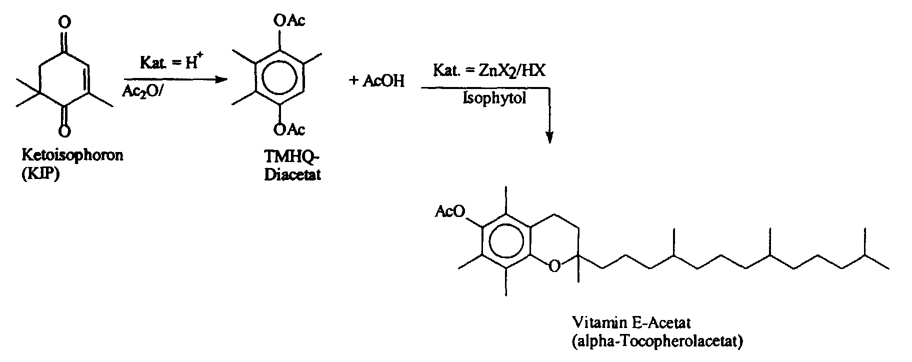

Dies zeigt, daß 2,3,5-Trimethylhydrochinondiester wertvolle Zwischenprodukte für die Herstellung von Chromanverbindungen sind.

### Stand der Technik

Die Produktion von KIP, dem Edukt der Trimethylhydrochinon-Diester Synthese erfolgt nach bekannten Verfahren z. B. durch die Sauerstoff-Oxidation von β-Isophoron mit anschließender destillativer Aufarbeitung (US-A 5 874 632).

In dem technischen Produkt sind je nach Isolierungsaufwand in unterschiedlichem Umfang Nebenprodukte enthalten. Dabei handelt es sich neben geringen Mengen von α-Isophoron hauptsächlich um 4-Hydroxy-Isophoron (HIP) und Lanieron. HIP und Lanieron verhalten sich bei der acylierenden Aromatisierung nicht inert, sondern bilden Folgeprodukte. So entstehen unter den Bedingungen der TMHQ-DA Synthese aus HIP diverse acylierte aromatische Verbindungen. Das Hauptprodukt der unter den Reaktionsbedingungen gefundenen Produkte wurde als 2,3,5-Trimethylphenolester identifiziert. Im folgenden Schema ist die Bildung des Nebenproduktes skizziert:

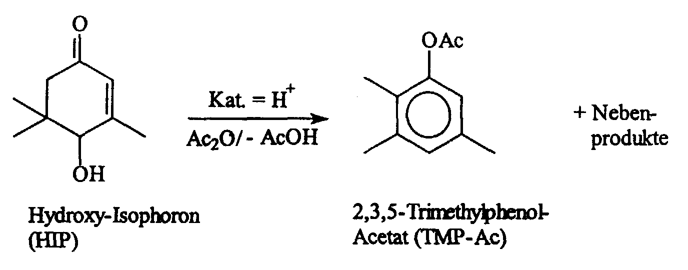

Lanieron, ein weiteres Nebenprodukt der β-IP Oxidation, das nur durch erheblichen destillativen Aufwand oder durch basische Reaktivextraktion von KIP zu trennen ist, reagiert unter den Bedingungen der Aromatisierung zu einem Gemisch, bestehend aus 3,4,5-Trimethylbrenzkatechindiester (3,4,5-TMBC-Diester)und dem 3,4,6-Trimethylbrenzkatechindiester (3,4,6-TMBC-Diester), wobei das 3,4,5-Isomer das Hauptprodukt der Umsetzung darstellt. Im folgenden Schema ist diese Aromatisierung von Lanieron mit einem Acylierungsmittel in Gegenwart eines sauren Katalysators (homogen oder heterogen) am Beispiel der Umsetzung mit Acetanhydrid skizziert:

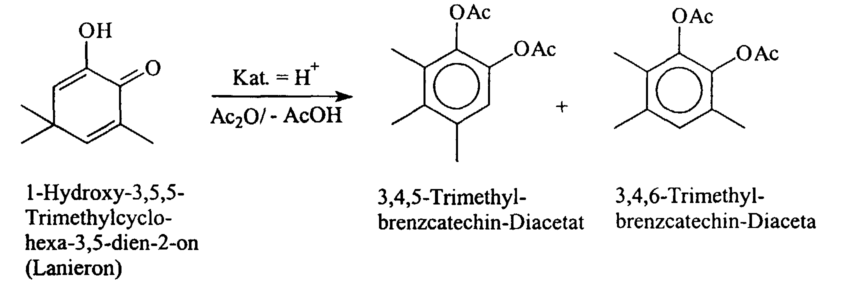

Bei der Aromatisierung von KIP, das kein Lanieron enthält, entsteht mit geeigneten Katalysatoren neben dem TMHQ-Diester nur der 3,4,5-TMBC-Diester als Nebenprodukt.

Insgesamt entsteht also beim Einsatz von technischem KIP (entweder aus β-Isophoron oder durch Direktoxidation aus alpha-Isophoron) als Edukt bei der Synthese von Trimethylhydrochinonestern ein Reaktionsprodukt, daß neben dem gewünschten Produkt diverse, oben angeführte Phenole und Brenzcatechine in Form ihrer Ester enthält. Ein Einsatz dieser Reaktionsrohmischung in der folgenden Kondensationsstufe mit Isophytol mit dem Ziel, reine Vitamin E-Ester zu synthetisieren, ist nicht möglich. Die beschriebenen Nebenprodukte sind unter den für die Kondensation üblichen Reaktionsbedingungen nicht inert, sondern reagieren ebenfalls mit Isophytol unter Wasseraustritt.Der so gebildete Vitamin E-Ester ist dann durch diverse Nebenprodukte verunreinigt, die sich nur aufwendig abtrennen lassen. Es erscheint vor diesem Hintergrund als vorteilhaft, die Nebenprodukte bereits auf der Stufe der Trimethylhydrochinon-Diester abzutrennen. Bisher gelang diese Abtrennung der Nebenprodukte (TMBC-Diester und TMP-Ester)nur durch aufwendige verfahrenstechnische Operationen, deren Nachteile wir im folgenden explizit ausführen.

Die Herstellung von 2,3,5-Trimethylhydrochinondiestern (TMHQ-Diester) aus Ketoisophoron (4-Oxo-Isophoron = KIP) in Gegenwart eines sauren Katalysators und eines Acylierungsmittels wie Carbonsäureanhydride, Acylhalogenide ist aus dem Stand der Technik bekannt (z. B. DE-A 2 149 159, EP 808 815 A2, EP 0 850 910 A1, EP 0 916 642 A1). Die Bildung der TMHQ-Diester aus KIP und einem Acylierungsmittel erfolgt in allen Fällen in Gegenwart eines sauren Katalysators oder eines Gemischs aus mehreren starken Säuren unter geeigneten Bedingungen. Die Reaktion selbst wird in vorteilhafter Weise so durchgeführt, daß Katalysator und Acylierungsmittel vorgelegt werden und KIP anschließend bei moderaten Temperaturen zugegeben wird. Man erhält üblicherweise nach Reaktionsende ein Gemisch der entsprechenden Trimethylhydrochinondiester, den oben angeführten Nebenprodukten, im wesentlichen aromatischen Verbindungen, überschüssigem Acylierungsmittel, dem Katalysator und Carbonsäure, das zur Isolierung des TMHQ-Diesters entsprechend aufgearbeitet werden muß.

Üblicherweise führt man die Reaktion nicht in einem zusätzlichen Lösungsmittel durch, sondern arbeitet in einem Gemisch (im Fall der Verwendung von Acetanhydrid als Acylierungsmittel) von Acetanhydrid und Essigsäure. Acetanhydrid, das üblicherweise in einem Überschuß eingesetzt wird, stellt in diesem Fall ein Reaktivlösungsmittel dar.

Die Isolierung aus der Reaktionslösung und Reinigung von Nebenprodukten erfolgt nach unterschiedlichen Methoden.

Aus der DE-A-2 149 159 ist ein Verfahren bekannt, bei dem man die Reaktionslösung mit Benzol extrahiert und nach der Verdünnung mit Ether aus Hexan verlustreich kristallisieren läßt. Das bedeutet auch, daß mindestens 3 Lösungsmittel zusätzlich eingebracht werden, die in zusätzlichen Schritten wieder zurückgewonnen werden müssen.

Es ist ebenfalls bekannt, daß nach Neutralisation des stark sauren Katalysators der gewünschten TMHQ-Diester durch Zugabe von Wasser oder einer Mischung aus Wasser und einem Lösungsmittel, vorzugsweise einer Carbonsäure, aus der Reaktionslösung auskristallisiert (EP-A 808 815). Nach dieser Verfahrensweise ist eine Wiederverwendung des Katalysators aufwendig oder nicht möglich. Auch die Wiedergewinnung oder Recyclierung von nicht verbrauchtem Acylierungsmittel, das gemäß dem Stand der Technik immer im Überschuß eingesetzt wird, ist nicht möglich, da es unter diesen Bedingungen hydrolysiert. Bei der gegebenenfalls eintretenden Hydrolyse des Diesters entsteht TMHQ, das zwar ebenfalls als Äquivalent von 2,3,5-Trimethylhydrochinon-Diacetat (TMHQ-DA) bei der Herstellung von Vitamin E-Acetat durch Kondensation mit Isophytol verwendet werden kann. Die im Vitamin E-Acetat vorhandene Acyleinheit (wesentlich für die Lagerstabilität dieser Vitamin E Vermarktungsform) muß bei der Verwendung von TMHQ anstelle von TMHQ-DA dann nachträglich eingeführt werden. Die Isolierung von TMHQ-DA in wässrigen Medien ist deshalb nicht wünschenswert und letztlich unwirtschaftlich.

### Aufgabe:

Es ist Aufgabe der vorliegenden Erfindung, die oben skizzierte aufwendige Verfahrensweise wesentlich zu vereinfachen und damit ein wirtschaftliches Verfahren zu finden.

Insbesondere stellte sich die Aufgabe, ein Verfahren zu finden, mit dem man aus KIP, wie es als technische Ware verfügbar ist, TMHQ-Diester herstellen und so isolieren kann, daß diese ohne weitere Verfahrensschritte wie Reinigen und Trocknen zum Einsatz bei der Synthese der Chromanverbindungen geeignet sind.

### Lösung:

Die Erfindung betrifft ein Verfahren zur Herstellung von veresterten Chromanverbindungen, bei dem man
1.1 in technischer Reinheit vorliegendes Ketoisophoron (KIP) mit einem Acylierungsmittel in Gegenwart einer Protonensäure zu einem Trimethylhydrochinoneser (TMHQ-Ester) umsetzt und
1.2 diesen Ester anschließend mit einem Allylalkoholderivat oder einem Allylalkohol in Gegenwart von Zinkhalogeniden und Protonen abspaltenden Säuren zu Reaktion bringt,
   dadurch gekennzeichnet, daß man
   1.1.1. die in Reaktionsschritt 1.1 gewonnene Lösung auf eine Temperatur zwischen 5 und 40°C abkühlt,
   1.1.2. das auskristallisierte Produkt abtrennt und wäscht,
   1.1.3. das bei der Abtrennung und gegebenenfalls das bei der Wäsche erhaltene Filtrat als Lösungsmittel für den Folgeansatz gemäß Reaktionsschritt 1.1 einsetzt,
   1.2.1 das gegebenenfalls gewaschene Produkt ohne Trocknung in die Reaktion gemäß 1.2 einführt und
   1.2.2 das gewünschte Produkt gegebenenfalls nach weiterer Acylierung isoliert.

Aus der Reaktionslösung von KIP und einem Acylierungsmittel kristallisiert nach erfolgter Umsetzung ein Teil des gebildeten TMHQ-Diesters bei dem Abkühlen der Reaktionslösung in hoher Reinheit aus, ohne daß die Zugabe eines Hilfsstoffes erforderlich ist.

Geeignete Acylierungsmittel sind Carbonsäureanhydride, wobei die Anhydride der Carbonsäuren mit 2 bis 5 Kohlenstoffen, insbesondere der Essigsäure bevorzugt sind.

Geeignete Katalysatoren sind stark saure anorganische oder organische Säuren, aber auch Mischungen daraus. Diese können entweder in heterogener Form (z.B. Zeolithe, Nafione oder stark saure Ionenaustauscher) oder in homogener Form (z.B. Sulfonsäuren, Borsäure / Oxalsäure oder Oleum) wirksam sein. Die Wahl des Katalysators ist für das Verfahren an sich kein wesentliches Kriterium, jedoch ist die Wirtschaftlichkeit stark von der erreichten Selektivität abhängig. Die ungelösten Katalysatoren sollen leicht abtrennbar sein und die gelösten Katalysatoren sollen von der Art und der Menge so gestaltet sein, daß sie auch bei den Temperaturen, bei denen die Produktabtrennung vorgenommen wird, in der Reaktionslösung vollständig gelöst vorliegen.

Für das erfindungsgemäße Verfahren ist als Einsatzstoff ein KIP in einer technischen Qualität mit Lanieron und HIP als Verunreinigungen geeignet.Deren Konzentrationen sind für die Durchführung des Verfahrens nicht kritisch, jedoch sollte aus wirtschaftlichen Überlegungen eine Obergrenze von 5 bis 10 Gew.-% nicht überschritten werden, da die Nebenprodukte im KIP Acylierungsmittel verbrauchen und die Ausbeute insgesamt verschlechtern.

Die bevorzugte Reaktionstemperatur ist weitgehend von dem ausgewählten Katalysator abhängig und kann in einem weiten Bereich zwischen 25 und 150°C oder darüber liegen. Das Acylierungsmittel wird bevorzugt in stöchiometrischer Menge, also 2 mol pro mol KIP, eingesetzt, kann aber auch im Unter- oder Überschuß zugesetzt werde. Dieser Unter- oder Überschuß ist für das erfindungsgemäße Verfahren nicht kritisch, da der nicht reagierte Anteil an Edukten unverändert und nahezu vollständig in den Prozeß zurückgeführt wird.

Nach erfolgter Umsetzung werden bei dem Einsatz von heterogenen Katalysatoren diese abgetrennt. Die weiteren Verfahrensschritte unterscheiden sich nicht von denen, die bei homogener Katalyse durchgeführt werden.

Die erhaltenen Reaktionslösungen, die aus dem TMHQ-Diester, der Carbonsäure, nicht abreagierten Edukten, den oben aufgeführten Nebenprodukten und gegebenenfalls dem Katalysator besteht, werden auf eine Temperatur zwischen 5 und 40°C, vorzugsweise 10 bis 30°C abgekühlt. TMHQ-Diester sind in diesem Temperaturbereich in der Reaktionslösung recht gut löslich, so daß nur ein geringer Teil des Produktes kristallisiert. Nach Abtrennen des Feststoffes erhält man als Filtrat eine bei der Filtrationstemperatur an TMHQ-Diester in der bei der Reaktion gebildeten Carbonsäure gesättigte Lösung. Diese enthält zusätzlich die nicht umgesetzten Edukte, Nebenprodukte und gegebenenfalls den Katalysator. Dieses Filtrat wird bei dem Folgeansatz bevorzugt als Lösungsmittel oder als Teil davon eingesetzt. KIP und Acylierungsmittel können jetzt bevorzugt in dem molaren Verhältnis von 1 : 2 eingesetzt werden.

Nach erfolgter Umsetzung wird die neu gebildete Carbonsäure, oder ein Teil davon, abdestilliert. Die Destillationsmenge wird bevorzugt so bemessen, daß das Volumen des Filtrats konstant bleibt. Durch Abkühlen der Reaktionslösung kristallisiert TMHQ-Diester aus, der abgetrennt und mit einem Teil der abdestillierten Carbonsäure, oder einer gesättigten Lösung des TMHQ-Diesters in dieser Carbonsäure gewaschen wird.

Die Menge der Waschflüssigkeit soll so bemessen sein, daß die gesamte anhaftende Reaktionslösung verdrängt wird, und ist von der Effizienz des Filtersystem abhängig. Das hierbei erhaltene Filtrat wird in den Kreislauf zurückgeführt.

Die Destillation der Carbonsäure kann wahlweise auch nach der Kristallisation und der Abtrennung des TMHQ-Diesters aus den Filtraten erfolgen.

Dieser Vorgang wird bevorzugt mehrmals wiederholt. Bei jedem Cyclus nimmt die Konzentration an Nebenprodukten in der im Kreis geführten Lösung zu. Um zu vermeiden, daß sich diese Nebenprodukte so stark anreichern, daß sie mit den TMHQ-Diestern kristallisieren oder in die Kristalle eingeschlossen werden, muß die Konzentration an Nebenprodukten in der im Kreis geführten Lösung unterhalb gewisser Höchstgrenzen bleiben. Diese liegt bei etwa 20 Gew.-% bezogen auf die beiden Brenzkatechindiestern im Filtrat. Das wird dadurch erreicht, daß ein Teil des Filtrats aus dem Kreislauf genommen wird. Dieser Teil soll so bemessen sein, daß darin die Menge an Nebenprodukten gelöst ist, die bei einem Umlauf neu gebildet wird. Sobald ein Teil des Filtrates nicht mehr zurückgeführt wird, muß der in diesem Teil enthaltene Anteil an Acylierungsmittel und gegebenenfalls auch der des Katalysators ergänzt werden. Die Destillationsmenge der Carbonsäure wird verringert, so daß weiterhin die im Kreis geführte Filtratmenge konstant bleibt.

Dieses Verfahren kann sowohl kontinuierlich, als auch diskontinuierlich oder in einer Kombination dieser Vorgehensweisen betrieben werden.

Man erhält so einen Filterkuchen, der ohne weitere Verfahrensschritte wie z. B. Umkristallisation, Hydrolyse oder Trocknung bei der Chromanderivatsynthese, insbesondere der Vitamine E-Acetat-Synthese vorteilhaft eingesetzt werden kann.

Der Filterkuchen enthält bzw. besteht bevorzugt zu 10 bis 50 Gew.-% aus der dem Acylierungsmittel entsprechenden Carbonsäure, 50 bis 90 Gew.-% aus dem TMHQ-Diester dieser Säure, 0,001 bis 2 Gew.-% aus einer Mischung isomerer Brenzkatechindiester und 0,001 bis 2 Gew.-% einer Mischung aus Trimethylphenolester, Katalysator und eingesetztem Acylierungsmittel. Der Anteil an Carbonsäure kann ohne Nachteil über dem oben genannten Wert liegen, da es gelungen ist, die in Schritt 1.1 als Nebenprodukt gebildete Carbonsäure bei der Synthese von Chromanderivaten, insbesondere α-Tocopherolestern als Co-Lösungsmittel einzusetzen. Auch die bei dem Aufkonzentrieren des Filtrats durch Destillation erhaltene Carbonsäure kann hier gegebenenfalls eingesetzt werden.

Für die anschließende Kondensation wird der auf die oben beschriebene Weise erhaltene Filterkuchen, der bei Verwendung von Acetanhydrid hauptsächlich TMHQ-DA neben Essigsäure enthält, ohne weitere Vorbehandlung eingesetzt. Üblicherweise legt man das essigsaure TMHQ-DA vor und suspendiert oder löst den Feststoff in einem geeigneten Lösungsmittel. Anschließend gibt man sukkzessive das Zinkhalogenid und die entsprechende bevorzugt wasserhaltige Protonensäure als Katalysatorkomponente zu.

Sollte die Brönstedtsäure nicht in einer wässrigen Form vorliegen, muß zusätzlich noch Wasser zu der vorgelegten Mischung dosiert werden. Die Reihenfolge der Zugabe der Komponenten an dieser Stelle ist nicht kritisch und kann variiert werden.

Erfindungsgemäß stellt man insbesondere α-Tocopherolestern, seine Derivate oder -homologe gemäß der allgemeinen Formel 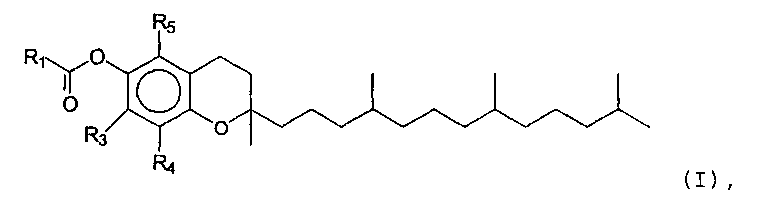 her. Bei diesem Verfahren bringt man Mono- oder Diester eines Hydrochinons insbesondere den Diester gemäß der allgemeinen Formel 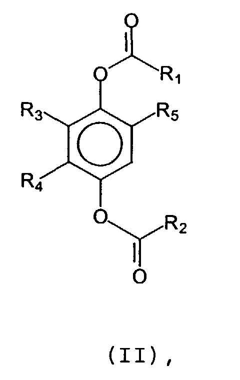 in der bedeuten:
- R₁, R₂:: Alkyl mit C₁-C₅, verzweigt oder unverzweigt, gesättigt oder ungesättigt, insbesondere Ethyl,
- R₃, R₄, R₅:: H, Alkyl mit C₁-C₃, gleich oder verschieden, insbesondere Methyl
mit einem Allylalkoholderivat der allgemeinen Formel 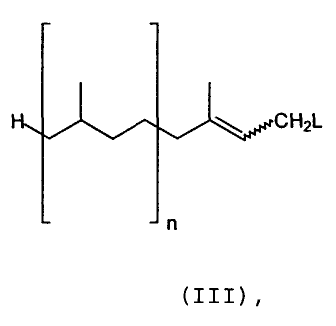 in der n eine Zahl von 0 bis 5 und L einer Hydroxyl-, Halogen-, Acetoxy-, Methansulfonyloxy-, Ethansulfonyloxy-, Benzolsulfonyloxy- oder Toluolsulfonylgruppe entspricht,
oder mit einem Allylalkohol der allgemeinen Formel 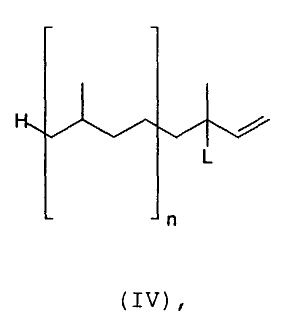 in der n dieselben Zahlen repräsentiert wie oben und L einer Hydroxyl-, Halogen- oder Acetoxygruppe entspricht, in Gegenwart von Zinkhalogeniden und Protonen abspaltenden Säuren bei einer Temperatur von 25 bis 100 °C zur Reaktion.

Zu dieser Mischung gibt man bevorzugt Isophytol über eine geeignete Dosiervorrichtung zu. Das im Reaktionsgemisch vorhandene Wasser und die über das Edukt im Reaktionssystem vorhandene und bei der Kondensation des Diacetats sich bildende Essigsäure verbleiben im Reaktionsgemisch und werden nicht ausgeschleust. Nach Ablauf der Reaktion erhält man einen nahezu vollständigen Umsatz der Eduktkomponenten TMHQ-DA und Isophytol, während das Katalysatorsystem nicht oder nur unwesentlich verbraucht ist. So hergestellte Chromanderivate, insbesondere nicht verestertes Vitamin E, werden im Anschluß an die Umsetzung nach bekannten Verfahren gegebenenfalls in die als Produkt gewünschten Ester, insbesondere das Vitamin E-Acetat, überführt. Hierbei kann man z. B. sowohl nach der Kondensationsreaktion direkt mit Acetanhydrid und einem geeigneten Katalysator verestern, oder man trennt zunächst durch Extraktion den sauren Katalysator ab und verestert anschließend.

Trotz der Gegenwart von Wasser und eines sauren Milieus, das eigentlich die Verseifung fördern sollte, werden die gewünschten Ester (Vitamin E-Acetat) aber bereits direkt bei der Kondensation in hoher Ausbeute neben unverestertem Vitamin E erhalten. Besonders bewährt hat sich die Verfahrensweise , Wasser in Konzentrationen von 10⁻² bis 200 Mol% bezogen auf TMHQ-DA in der Reaktionsmischung einzustellen. Das kann über die Dosierung der wässrigen Protonensäure erfolgen, oder aber über direkte Zugabe von Wasser zur Reaktionsmischung. Bei Verwendung von Zinkhalogeniden bietet sich die Verwendung der entsprechenden Halogenwasserstoffsäure an. Die Umsetzung wird vorteilhaft mit einer Mischung von Zinkchlorid/HCl oder Zinkbromid/HBr katalysiert; die Katalyse durch die gemischten Katalysatorsysteme, z.b. ZnBr₂/HCl ist aber ebenfalls geeignet. Als Protonensäuren (Brönstedtsäuren) sind aber auch geeignet: Schwefelsäure, Schwefelsäure/SO₃-Mischungen mit verschiedenen SO₃ Gehalten, entsprechende Supersäuren mit einem H₀-Wert ≤ - 11,9, wie z.B. Trifluormethansulfonsäure, Halogensulfonsaure, Perhalogensäuren, Borsäure-Schwefelsäure Mischungen und Katalysatoren, die als Komponente Bis(trifluormethansulfonyl)amin oder entsprechende Metallsalze des Amins enthalten mit der allgemeinen Formel Me[N[SO₃CF₃]₂]n, wobei Me ein Metall repräsentiert und n die Valenz des entsprechenden Metalls. Als Protonensäuren sind insbesondere auch Gemische aus Borsäure einerseits und Oxalsäure andererseits in einem molaren VerVerhältnis von 1:1 bis 1:5, besonders 1:2 geeignet. Die Verwendung der Protonensäuren erfolgt in einem Konzentrationsbereich von 10⁻² Mol% bis 100 Mol% bezogen auf eingesetztes TMHQ-DA, während die Lewissäuren in einem Konzentrationsbereich von 10-100 Mol% bezüglich TMHQ-DA eingesetzt werden können. Auch größere Katalysatormengen sind für die Durchführung geeignet,eine Erhöhung der Katalysatormengen bringt aber keinen weiteren wirtschaftlichen Vorteil.

Wenn auch keine besonderen Beschränkungen im Hinblick auf die Menge des eingesetzten Lösungsmittels vorhanden sind, arbeitet man bevorzugt mit einer Lösungsmittelmenge von 0,05 bis 100 g/g eingesetztem TMHQ-DA, wobei 0,1-10g /g TMHQ-DA besonders bevorzugt sind. Geeignete organische Lösungsmittel für die Umsetzung sind die aus der EP-A-0 924 208 bekannten Carbonsäureester, inclusive Carbonatester, zu denen gehören
Dimethylcarbonat,
Diethylcarbonat,
Dipropylcarbonat,
Methylethylcarbonat,
Ethylencarbonat und
Propylencarbonat
oder Carbonsäureester, wie z. B.
n-Propylacetat,
i-Propylacetat,
n-Butylacetat,
i-Butylacetat,
t-Butylacetat,
n-Amylacetat,
i-Amylacetat [CH₃COOCH₂CH₂CH(CH₃)₂],
sec-Amylacetat [CH₃COOCH(CH₃)CH₂CH₂CH₃],
t-Amylacetat [CH₃COOC(CH₃)₂CH₂CH₃],
2,2-Dimethylpropylacetat [CH₃COOCH₂C(CH₃)₃],
2-Methylbutylacetat [CH₃COOCH₂CH(CH₃)CH₂CH₃],
Methylpropionat,
n-Butylpropionat,
Ethylbutyrat,
i-Propylbutyrat,
Methylisobutyrat,
Ethylisobutyrat,
i-Butylisobutyrat,
Methylvalerat,
Ethylvalerat,
Methylisovalerat,
Ethylisovalerat,

Besonders bevorzugt sind n-Propylacetat, i-Propylacetat, n-Butylacetat, i-Butylacetat, n-Butylpropionat, Ethylbutyrat, i-Propylbutyrat, Methylisobutyrat, Ethylisobutyrat, Methylvalerat,
oder nicht polare Lösungsmittel wie z. B.
Pentan, Hexan, Heptan, Octan,
Ligroin, Petrolether, Cyclohexan,
Benzol, Toluol und Xylol.
oder aliphatische Alkohole, wie z. B.
Methanol,
Ethanol,
n-Propanol,
i-Propanol,
n-Butanol,
i-Butanol,
t-Butanol,
n-Amylalkohol (1-Pentanol),
2-Pentanol (1-Methyl-1-Butanol)
3-Pentanol (1-Ethyl-1-Propanol)
i-Amylalkohol (3-Methyl-1-Butanol)
t-Amylalkohol (1,1-Dimethyl-1-Propanol)
2,2-Dimethyl-1-Propanol,
1,2-Dimethyl-1-Propanol,
2-Methyl-1-Butanol, und
3-Methyl-2-Butanol.

Besonders bevorzugt sind n-Propanol, i-Propanol, n-Butanol, i-Butanol, t-Butanol, n-Amylalkohol, 2-Pentanol, 3-Pentanol, i-Amylalkohol und t-Amylalkohol, sowie Gemischen aus den genannten Gruppen von Lösungsmitteln.In Gemischen wirkt eines der Lösungsmittel als Cosolvens.

Einen besonderen Einfluß auf die Selektivität findet man bei Verwendung von cyclischen Carbonaten wie unter anderem Ethylen- oder Propylencarbonat, offenkettigen Estern der Essigsäure wie Ethylacetat, Propylacetat, Butylacetat und Isobutylacetat.

Als Co-Lösungsmittel können nach der vorliegenden Erfindung die bei der TMHQ-DA Herstellung verwendeten, mit dem Acylierungsmittel korrespondieren, organischen Carbonsäuren, im einfachsten Fall (bei Verwendung von Acetanhydrid bei der Umsetzung von KIP) Essigsäure, eingesetzt werden.

Die Reaktion kann sowohl kontinuierlich als auch diskontinuierlich durchgeführt werden.

Durch den Einsatz eines essigsauren, Nebenkomponenten enthaltenden TMHQ-DA ist die gebräuchliche Verfahrensweise erheblich vereinfacht worden. Die im Edukt enthaltene Essigsäure fällt auch bei der Acetylierung zu Vitamin E-Acetat an und muß daher nur an dieser Stelle aus dem Produkt entfernt werden. Damit ist es insbesondere gelungen, eine Tocopherolacetatsynthese inclusive der Produktion von TMHQ-DA als technische Intermediat aus KIP durch Verringerung der verfahrenstechnischen Operationen bei der Eduktherstellung wesentlich zu vereinfachen.

Durch die folgenden Beispiele soll das erfindungsgemäße Verfahren veranschaulicht werden, ohne daß diese einschränkend wirken sollen.

### Beispiele

### Beispiel 1

In einen 2-Liter-Rührkolben wird 11 Filtrat aus einem vorangegangenen Versuch eingefüllt. Dieses Filtrat enthält:

| | |
|---|---|
| TMHQ-Diacetat | 32,6% |
| 3,4,5-TMBC-Diacetat | 16,9% |
| 3,4,6-TMBC-Diacetat | 1,9% |
| 2,3,5-Trimethylphenolacetat | 1,3% |
| Trifluormethansulfonsäure | 0,9% |
| Essigsäure | 37,7% |
| Essigsäureanhydrid | 8,4% |

215 g Essigsäureanhydrid und 450 mg Trifluormethansulfonsäure werden zugegeben. 156,2 g KIP mit einem Reingehalt von 97,3 % werden bei einer Temperatur von 50 bis 55 °C innerhalb von 20 Minuten zugeführt. Weitere 2 Stunden wird die Temperatur in diesem Bereich gehalten. Die braun gefärbte Reaktionslösung wird in eine Destillationsapparatur überführt und bei 35 mbar werden 110 g Essigsäure abdestilliert. Die zurückbleibende Lösung wird bis auf 20 °C abgekühlt, wobei das Produkt auskristallisiert. Kristallisation eintritt. Die Suspension wird 1 Stunde gerührt, danach werden die Kristalle über eine Nutsche abgetrennt. Die Kristalle werden angedrückt und mit 90 g einer gesättigten Lösung von TMHQ-Diacetat in Essigsäure gewaschen. 55 g des Filtrats werden entnommen, die verbleibende Menge wird bei dem nächsten Ansatz wieder eingesetzt.

Der Filterkuchen wiegt 301 g und hat folgende

| | | |
|---|---|---|
| Zusammensetzung: | TMHQ-Diacetat | 77,2 % |
| | Essigsäure | 22,4 % |
| | Essigsäureanhydrid | 0,07 % |
| | 3,4,5-TMBC-Diacetat | 0,18 % |
| | 3,4,6-TMBC-Diacetat | 0,02 % |
| | 2,3,5-Trimethylphenolacetat | 0,01 % |
| | Trifluormethansulfonsäure | 0,01 % |

Nach Abzug der bei dem Waschvorgang zugegebenen Menge an TMHQ-Diacetat beträgt die isolierte Ausbeute 87%. Dieser Filterkuchen kann ohne weitere Behandlung bei der Umsetzung zu VitaminE-Acetat eingesetzt werden.

### Beispiel 2

Das aus Beispiel 1 stammende essigsaure TMHQ-DA, das die genannten Brenzcatechin-Diester und Phenolester enthält, wird mit Isophytol zur Herstellung von Vitamin E-Acetat umgesetzt:

77,6 g des TMHQ-DA Filterkuchens aus Beispiel 1 (enthält 59,9 g = 250 mmol TMHQ-DA, 17,7 g Essigsäure + Nebenprodukte) wird in 90 ml Toluol suspendiert. Zu dieser Suspension gibt man 40 Mol% ZnBr₂ und 18 Mol% wässrige, konzentrierte HBr. Die Reaktionsmischung wird auf 60°C erwärmt und innerhalb von 4 h werden über eine geeignete Pumpe 105 Mol% Isophytol zudosiert. Nach beendeter Zugabe wird noch weitere zwei Stunden nachgerührt. Nach Beendigung der Reaktion kühlt man auf Raumtemperatur ab, wobei sich zwei Phasen bilden. Die untere, im wesentlichen aus Katalysator, Wasser und Essigsäure bestehende Phase wird abgetrennt. Die obere Phase enthält ein Gemisch von Vitamin E neben Vitamin E-Acetat im Verhältnis 34: 66 (Quantifizierung per HPLC). Man erwärmt die organische Phase auf 40°C und dosiert 130 Mol% Acetanhydrid (bez. auf Vitamin E Konzentration) zu dieser Lösung, um freies Vitamin E zu Vitamin E-Acetat umzusetzen. Nach einer Reaktionszeit von 2 h wird auf Raumtemperatur abgekühlt. Man gibt zu der erhaltenen Reaktionslösung 200 ml Toluol und wäscht die Lösung mit 2 x 30 ml Wasser und anschließend mit 30 ml gesättigter NaHCO₃-Lösung. Man trennt die organische Phase ab, trocknet über Magnesiumsulfat und zieht nach Filtration des Salzes das Lösungsmittel am Rotationsverdampfer ab. Nach HPLC Quantifizierung des erhaltenen Rohöls ergibt sich eine Vitamin E-Acetat Ausbeute von 95,6% bezogen auf TMHQ-DA (d.h. 91,0% bez. auf Isophytol).

## Patentansprüche

1. Verfahren zur Herstellung von veresterten Chromanverbindungen, bei dem man
1.1 in technischer Reinheit vorliegendes Ketoisophoron (KIP) mit einem Acylierungsmittel in Gegenwart einer Protonensäure zu einem Trimethylhydrochinonester (TMHQ-Ester) umsetzt und
1.2 diesen Ester anschließend mit einem Allylalkoholderivat oder einem Allylalkohol in Gegenwart von Zinkhalogeniden und Protonen abspaltenden Säuren zu Reaktion bringt, und
1.3 gegebenenfalls in Gegenwart eines organischen Lösungsmittels arbeitet
**dadurch gekennzeichnet**,
daß man
1.1.1 die in Reaktionsschritt 1.1 gewonnene Lösung auf eine Temperatur zwischen 5 und 40°C abkühlt,
1.1.2 das auskristallisierte Produkt abtrennt und wäscht,
1.1.3 das bei der Abtrennung und gegebenenfalls das bei der Wäsche erhaltene Filtrat als Lösungsmittel für den Folgeansatz gemäß Reaktionsschritt 1.1 einsetzt,
1.2.1 das gegebenenfalls gewaschene Produkt ohne Trocknung in die Reaktion gemäß 1.2 einführt und
1.2.2 das gewünschte Produkt gegebenenfalls nach weiterer Acylierung isoliert.

2. Verfahren gemäß Anspruch 1,
**dadurch gekennzeichnet**,
daß man in Stufe 1.2 den Mono- oder Diester eines Hydrochinons gemäß der allgemeinen Formel 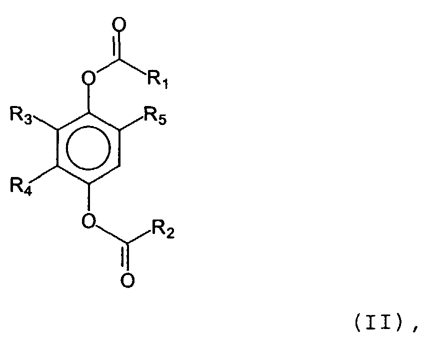 in der bedeuten:
R₁, R₂: Alkyl mit C₁-C₅, verzweigt oder unverzweigt,gesättigt oder ungesättigt
R₃, R₄, R₅: H, Alkyl mit C₁-C₃, gleich oder verschieden,
mit einem Allylalkoholderivat der allgemeinen Formel 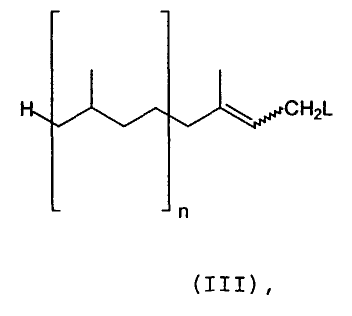 in der n eine Zahl von 0 bis 5 und L einer Hydroxyl-, Halogen-, Acetoxy-, Methansulfonyloxy-, Ethansulfonyloxy-, Benzolsulfonyloxy- oder Toluolsulfonylgruppe entspricht,
oder mit einem Allylalkohol der allgemeinen Formel 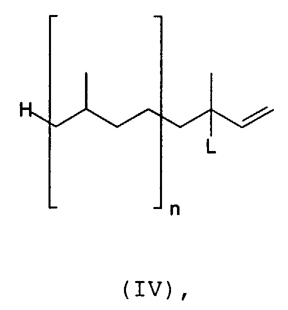 in der n dieselben Zahlen repräsentiert wie oben und L einer Hydroxyl-, Halogen- oder Acetoxygruppe entspricht,bei einer Temperatur von 25 bis 150°C zur Reaktion bringt.

3. Verfahren gemäß den Ansprüchen 1 und 2,
**dadurch gekennzeichnet**,
daß man in Schritt 1.2.1 TMHQ-Diacetat und Isophytol einsetzt.

4. Verfahren gemäß Anpruch 1,
**dadurch gekennzeichnet**,
daß man in Schritt 1.2.1 einen Filterkuchen einsetzt, enthaltend, bezogen auf die Gesamtmenge,
10 bis 50 Gew.-% der dem Acylierungsmittel entsprechenden Carbonsäure, insbesondere Essigsäure,
50 bis 90 Gew.-% TMHQ-Diester,
0,001 bis 2 Gew.-% einer Mischung isomerer Brenzkatechindiester, und
0,001 bis 2 Gew.-% einer Mischung aus Trimethylphenolester, Katalysator und nicht umgesetztem Acylierungsmittel.

5. Verfahren gemäß Anspruch 1,
**dadurch gekennzeichnet**,
daß man die während der Reaktion gemäß Schritt 1.1 entstandene Carbonsäure zumindest zum Teil abdestilliert und gegebenenfalls in eine der Stufen 1.1 oder 1.2 zurückführt.

6. Verfahren gemäß Anspruch 5,
**dadurch gekennzeichnet**,
daß man die während der Reaktion entstandene Carbonsäure vor oder nach dem Auskristallisieren des TMHQ-Diesters abdestilliert und gegebenenfalls in eine der Stufen 1.1 oder 1.2 zurückführt.

7. Verfahren gemäß Anspruch 1,
**dadurch gekennzeichnet**,
daß man das Verfahren kontinuierlich durchführt.
